# EUROPEAN PATENT APPLICATION

(11) **EP 4 742 259 A1**
(43) Date of publication of application: **13.05.2026**
(21) Application number: 25213819.3
(22) Date of filing: 06.11.2025
(51) Int. Cl.: G16H 20/10, G16H 50/20, G16H 50/30, G16H 70/40

(54) **METHOD AND DEVICE FOR PREDICTING THE APPROPRIATENESS OF ANTIBIOTIC USE IN PATIENTS WITH INFECTIOUS DISEASES**

(30) Priority: 06.11.2024 KR 20240156550; 01.07.2025 KR 20250088038
(71) Applicant: INDUSTRY-ACADEMIC COOPERATION FOUNDATION, YONSEI UNIVERSITY, Seoul 03722 (KR); Industry-University Cooperation Foundation Hanyang University, Seoul 04763 (KR)
(72) Inventor: Lee, Yong Seop, Seoul (KR); Heo, Seok Jae, Seoul (KR); Ku, Nam Su, Gyeonggi-do (KR); Park, Se Yoon, Gyeonggi-do (KR)
(74) Representative: Müller Schupfner & Partner Patent- und Rechtsanwaltspartnerschaft mbB (Muc)

(57) **Abstract**

The present invention relates to a method for predicting the appropriateness of antibiotic use in a patient with an infectious disease and a device thereof, comprising receiving a bio-signal data, blood test data, and clinical characteristic data for a subject; and predicting appropriateness of antibiotic therapy for the subject based on the received bio-signal data, blood test data, and clinical characteristic data using a prediction model configured to predict the appropriateness of antibiotic use with the bio-signal data, blood test data, and clinical characteristic data as input.

## Description

### CROSS-REFERENCE TO RELATED APPLICATIONS

This application claims the priority of Korean Patent Application No. 10- 2024-0156550 filed on November 6, 2024 and 10-2025-0088038 filed on July 1, 2025, in the Korean Intellectual Property Office, the disclosure of which is incorporated herein by reference.

### BACKGROUND

### Field

The present invention relates to a method for predicting the appropriateness of antibiotic use in a patient with an infectious disease and a device thereof.

### Description of the Related Art

Bacteremia can refer to the clinical condition in which the bacteria enter the blood and circulate throughout the body, and bloodstream infection is a synonym. This bacteremia can occur when bacteria proliferate at a rate that exceeds the removal capacity of the reticuloendothelial system(RES).

At this time, bacteremia can be classified into transient bacteremia, intermittent bacteremia, and persistent bacteremia according to clinical manifestations. More specifically, transient bacteremia is bacteremia that occurs in the early stages of systemic or local infection and may be accompanied by meningitis, pneumonia, purulent arthritis, osteomyelitis, gonococcal or meningococcal infection. In addition, intermittent bacteremia can occur when there is an abscess in areas such as the abdominal cavity, pelvic cavity, perirenal region, liver, and prostate, and persistent bacteremia can occur when there is bacterial endocarditis and an infection of intravascular catheter, and can be accompanied by typhoid and brucellosis.

On the other hand, there may be the use of antibiotics as a treatment of bacteremia, and empirical use of appropriate antibiotics in the early stages can prevent the progression of bacteremia to sepsis and septic shock.

A description of the background is written to facilitate understanding of the invention. The matters described in this section should not be construed as admitted prior art.

### SUMMARY

On the other hand, in order to treat of bacteremia it is most effective to clearly identify the causative bacteria that caused the infection and select antibiotics suitable for the bacteria as the types of bacteria that cause human infection are also very diverse, but the blood culture test used to identify bacteria in samples collected from blood or infected tissues takes at least 2 to 7 days, so empirical antibiotics are treated in consideration of the patient's infection diagnosis and clinical symptoms before the exact causative bacteria are identified.

Furthermore, when a bacterial infection occurs in the body, an inflammatory reaction due to an excessive immune reaction occurs, and the symptoms rapidly deteriorate in a short time as the bacteria spread throughout the body through the bloodstream, so it is very important to treat appropriate antibiotics in the early stages of infection.

However, since pathogenic organism have not been precisely identified, there are many cases where two to three antibiotics are used simultaneously in the empirical antibiotic treatment mentioned above. Excessive antibiotic combination can harm patients, and the use of inappropriate antibiotics has the problem of promoting antibiotic resistance that causes bacteria to become resistant to antibiotics.

The inventors of the present invention have noted that hemodynamic indicators and various biomarker levels show different patterns depending on the appropriateness of empirical antibiotics as a way to overcome the aforementioned limitations.

Accordingly, the inventors used bio-signal data, blood test data, and clinical characteristic data on a blood-infected subject to which an appropriate antibiotic is administered and a blood-infected subject to which an inappropriate antibiotic is administered as training data to build a model that can predict the appropriateness of antibiotic use even before test results identifying bacteria present in a sample of a bacteremia patient in the early stages of the infection are available.

As a result, the inventors have developed a system for providing information on the appropriateness of antibiotic use for treating a patient with bacteremia in the early stage of a new infection based on a prediction model.

Accordingly, the inventors expected that the appropriateness of antibiotic therapy could be predicted through the treatment response of antibiotic therapy before the results of the bacterial identification test of patients with bacteremia were reported by the prediction model, so that medical staff could effectively treat bacteremia in the early stages of infection.

Accordingly, an object of the present invention is to provide a method for providing information on the appropriateness of antibiotic use and a device using the same, which are configured to predict the appropriateness of antibiotic therapy through a treatment response of antibiotic therapy for the subject based on bio-signal data, blood test data, and clinical characteristic data obtained from the subject using a prediction model.

The objects of the present disclosure are not limited to the objects mentioned above, and other objects, which are not mentioned above, will be apparent to those skilled in the art from the following descriptions.

To achieve the above-mentioned object, a method for providing information on the appropriateness of antibiotic use implemented by a processor according to an embodiment of the present invention is provided, the method comprising,
receiving bio-signal data, blood test data, and clinical characteristic data for a subject; and
in a first prediction step, predicting appropriateness of antibiotic therapy for said subject based on the received bio-signal data, blood test data, and clinical characteristic data using a prediction model configured to predict the appropriateness of antibiotic use with said bio-signal data, blood test data, and clinical characteristic data as input,
wherein said bio-signal data, blood test data, and clinical characteristic data for the subject are data obtained at a first time point,
wherein the first time point is within 5 days after antibiotic treatment,
wherein the appropriateness of antibiotic therapy for the subject in the first prediction step is defined as the appropriateness for the first time point.

According to a feature of the present invention, the subject may be a patient with bacteremia.

According to another feature of the present invention, the bacteremia may be a disease caused by at least one member selected from the group consisting of the species Staphylococcus aureus, the genus Streptococcus, the genus Enterococcus, the order Enterobacterales, the genus Acinetobacter, and the genus Pseudomonas.

According to another feature of the present invention, the bio-signal data and the blood test data may be time-series data collected from the subject for 5 days after administration of antibiotics.

According to another feature of the present invention, the bio-signal data may include at least one of a systolic blood pressure (SBP), a diastolic blood pressure (DBP), a body temperature, a heart rate, and a respiratory rate, and the bio-signal data may include a baseline value before taking an antibiotic and a value obtained at a time point within 5 days after taking the antibiotic in pairs.

According to another feature of the present invention, the blood test data may include at least one of an aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels, and the blood test data may include a baseline value before taking an antibiotic and a value obtained at a time point within 5 days after taking the antibiotic in pairs.

According to another feature of the present invention, the clinical characteristic data may include at least one of the following: age, gender (Sex), Charlson Comorbidity Index score (CCI_score), Pitt Score, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), cancer, or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicillin/clavulanate treatment status, or Ampicillin/sulbactam treatment status.

According to another feature of the present invention, the method further comprises re-receiving a bio-signal data, blood test data, and clinical characteristic data obtained at a second time point; and in a second prediction step, predicting the appropriateness of antibiotic therapy for the subject based on the re-received bio-signal data, blood test data, and clinical characteristic data, using the prediction model.

According to another feature of the present invention, the second time point is another time point between the first time point and 5 days after antibiotic treatment, and the appropriateness of antibiotic therapy for the subject in the second prediction step may be defined as appropriateness for the second time point.

According to another feature of the present invention, the re-received bio-signal data and blood test data may include a baseline value before administration of antibiotics and a value obtained at the second time point in pairs.

According to another feature of the present invention, the prediction model may include a first prediction model configured to predict appropriateness of antibiotic use using the bio-signal data, blood test data, and clinical characteristic data obtained at the first time point as input, and a second prediction model configured to predict appropriateness of antibiotic use using the bio-signal data, blood test data, and clinical characteristic data obtained at the second time point as input.

According to another feature of the present invention, the prediction model may be a model trained by steps of receiving training data consisting of bio-signal data, blood test data, and clinical characteristic data obtained from a blood-infected subject to which an appropriate antibiotic is administered and a blood-infected subject to which an inappropriate antibiotic is administered, and predicting the appropriateness of antibiotic use based on the training data.

According to another feature of the present invention, the prediction model may be any one selected from the group consisting of convolutional neural networks (CNNs), deep neural networks (DNNs), and recurrent neural networks (RNNs).

According to another feature of the present invention, the recurrent neural network (RNN) may be a long short-term memory (LSTM) or a gated recurrent unit (GRU).

To achieve the above-mentioned object, another embodiment of the present disclosure provides a device for providing information on the appropriateness of antibiotic use, comprising a receiving unit configured to receive bio-signal data, blood test data, and clinical characteristic data from a subject and a processor connected to the receiving unit.

According to a feature of the present invention, the processor is configured to predict the appropriateness of antibiotic therapy for the subject based on the received bio-signal data, blood test data, and clinical characteristic data, using a prediction model for appropriateness of antibiotic use configured to predict appropriateness of antibiotic use by taking bio-signal data, blood test data, and clinical characteristic data as inputs, wherein the bio-signal data, blood test data, and clinical characteristic data may be data obtained at a first time point, the first time point being within 5 days of antibiotic treatment, and the appropriateness of antibiotic therapy for the subject may be defined as appropriateness for the first time point.

According to another feature of the present invention, the subject may be a patient with bacteremia.

According to another feature of the present invention, the bio-signal data and blood test data may be time-series data collected from the subject for 5 days after administration of antibiotics.

According to another feature of the present invention, the bio-signal data may include at least one of a systolic blood pressure (SBP), a diastolic blood pressure (DBP), a body temperature, a heart rate, and a respiratory rate, and the bio-signal data may include a baseline value before taking an antibiotic and a value obtained at a time point within 5 days after taking the antibiotic in pairs.

According to another feature of the present invention, the blood test data may include at least one of an aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels, and the blood test data may include a baseline value before taking an antibiotic and a value obtained at a time point within 5 days after taking the antibiotic in pairs.

According to another feature of the present invention, the clinical characteristic data may include at least one of the following: age, gender (Sex), Charlson Comorbidity Index score (CCI_score), PittScore, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), cancer, or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicillin/clavulanate treatment status, or Ampicillin/sulbactam treatment status.

According to another feature of the present invention, the receiving unit is further configured to re-receive bio-signal data, blood test data, and clinical characteristic data obtained at a second time point from the subject, and the processor is further configured to predict the appropriateness of antibiotic therapy for the subject based on the re-received bio-signal data, blood test data, and clinical characteristic data, the second time point is another time point between the first time point and 5 days after antibiotic treatment, and the appropriateness of antibiotic therapy for the subject may be defined as appropriateness for the second time point.

According to another feature of the present invention, the prediction model for appropriateness of antibiotic use may include a first prediction model configured to predict appropriateness of antibiotic use using the bio-signal data, blood test data, and clinical characteristic data obtained at the first time point as input, and a second prediction model configured to predict appropriateness of antibiotic use using the bio-signal data, blood test data, and clinical characteristic data obtained at the second time point as input.

The effect according to the present disclosure is providing information associated with the appropriateness of antibiotic therapy by evaluating whether antibiotics treated for bacteremia patients are appropriate, by providing an information providing system based on an artificial neural network configured to predict appropriateness of antibiotic use using bio-signal data, blood test data, and clinical characteristic data.

Accordingly, present invention has the effect of predicting the appropriateness of antibiotic use with high reliability and accuracy, thereby enabling the optimal antibiotic treatment for a patient in the early stages of infection in the treatment of bacteremia, where early treatment is important.

More specifically, by providing an information providing system based on a prediction model trained to predict whether an antibiotic administered to a subject is an appropriate antibiotic, the appropriateness of antibiotic use for a patient with bacteremia can be predicted by the prediction model, thereby contributing to the effective treatment of medical staff even before the results of bacterial identification tests in a sample collected from blood or infected tissue are reported.

Accordingly, by providing a new information providing system, it is possible to predict the appropriateness of antibiotic therapy regardless of the skill level of the medical staff and provide highly reliable information thereon.

Furthermore, the present invention may provide an effect of increasing the survival rate of subjects with bacteremia and reducing the cost of preventing and treating complications.

The effects according to the present disclosure are not limited to the description exemplified above, and more various effects are included in the present specification.

### BRIEF DESCRIPTION OF THE DRAWINGS

FIG. 1A illustrates a system for providing information on the appropriateness of antibiotic use using a device for providing information on the appropriateness of antibiotic use according to an embodiment of the present invention.
FIG. 1B exemplarily illustrates a process of selecting an antibiotic based on a blood culture test to identify a pathogenic organism in a bacteremia patient.
FIG. 2A is a block diagram illustrating a configuration of an information providing server according to an embodiment of the present invention.
FIG. 2B is a block diagram illustrating a configuration of a medical staff device according to an embodiment of the present invention.
FIGS. 3A to 3C are flowcharts schematically illustrating a method of providing information on the appropriateness of antibiotic use according to an embodiment of the present invention.
FIG. 4A exemplarily illustrates a structure of a prediction model used in a method for providing information on the appropriateness of antibiotic use according to an embodiment of the present invention.
FIG. 4B illustrates a cell structure of a long short-term memory (LSTM) according to an embodiment of the present invention.
FIG. 5 illustrates a process of pre-processing and labeling training data of a prediction model for appropriateness of antibiotic use according to an embodiment of the present invention.
FIG. 6 exemplarily illustrates training data according to an embodiment of the present invention.
FIG. 7 illustrates the results of hemodynamic indicators and biomarker levels measured over time after antibiotic administration in patients administered appropriate antibiotics and patients administered inappropriate antibiotics, according to one embodiment of the present invention.
FIG. 8 illustrates an AUROC analysis result of a model for predicting the appropriateness of antibiotic use according to an embodiment of the present invention.

### DETAILED DESCRIPTION OF THE EMBODIMENT

Advantages and features of the present disclosure and methods of achieving the advantages and features will be clear with reference to embodiments described in detail below together with the accompanying drawings. However, the present disclosure is not limited to the embodiments disclosed herein but will be implemented in various forms. The embodiments of the present disclosure are just provided so that the present disclosure is completely disclosed, and a person with ordinary skill in the art to which the present disclosure pertains can fully understand the scope of the present disclosure. The present disclosure will be defined only by the scope of the appended claims. In connection with the description of the drawings, the similar reference numerals may be used for the similar components.

As used herein, the terms "have," "may have," "include," or "may include" indicate the existence of a feature (e.g., a number, function, operation, or a component such as a part) and do not exclude the existence of other features.

As used herein, the terms "A or B," "at least one of A and/or B," or "one or more of A and/or B" may include all possible combinations of A and B listed together. For example, "A or B," "at least one of A and B," "at least one of A or B" may indicate all of (1) including at least one A, (2) including at least one B, or (3) including at least one A and at least one B.

As used herein, the terms "first" and "second" may modify various components regardless of importance and do not limit the components. These terms are only used to distinguish one component from another. For example, a first user device and a second user device may indicate different user devices from each other regardless of the order or importance of the devices. For example, a first component may be named a second component, and similarly, the second component may also be named the first component, without departing from the scope disclosed in the present document.

It will be understood that when an element (e.g., a first element) is referred to as being (operatively or communicatively) "coupled with/to," or "connected to" another element (e.g., a second element), it can be coupled or connected with/to the other element directly or via a third element. In contrast, it will be understood that when an element (e.g., a first element) is referred to as being "directly coupled with/to" or "directly connected with/to" another element (e.g., a second element), no other element (e.g., a third element) intervenes between the element and the other element.

As used herein, the terms "configured (or set) to" may be interchangeably used with the terms "suitable for," "having the capacity to," "designed to," "adapted to," "made to," or "capable of" depending on circumstances. The term "configured (or set) to" does not essentially mean "specifically designed in hardware to." Rather, the term "configured to" may mean that a device can perform an operation together with another device or parts. For example, the term "processor configured (or set) to perform A, B, and C" may mean a generic-purpose processor (e.g., a central processing unit (CPU) or application processor) that may perform the operations by executing one or more software programs stored in a memory device or a dedicated processor (e.g., an embedded processor) for performing the operations.

The terms used in the present document are used to just describe a specific embodiment and do not intend to limit the scope of another embodiment. Singular expressions may include plural expressions unless clearly described as different meanings in the context. The terms used herein, including technical or scientific terms, may have the same meaning as commonly understood by those skilled in the art disclosed in the present document. The terms such as those defined in commonly used dictionaries may be interpreted as having meanings identical or similar to meanings in the context of related technologies and should not be interpreted as ideal or excessively formal meanings unless explicitly defined in the present document. In some instances, the terms defined in the present document should not be interpreted to exclude the embodiments disclosed in the present document.

Respective features of several embodiments of the present disclosure may be partially or entirely coupled to or combined with each other, and as sufficiently appreciated by those skilled in the art, various technical cooperation and operations may be made, and the respective embodiments may be carried out independently of each other or carried out together correlatively.

For clarity of the interpretation of the present specification, terms used in the present specification will be defined below.

As used herein, the term "subject" may mean any subject for whom information regarding the appropriateness of antibiotic use is sought.

For example, the subject may have bacteremia or be suspected of having bacteremia. Preferably, the subject for whom the appropriateness of antibiotic therapy is to be predicted may be one with acute severe bacteremia or one suspected of having acute severe bacteremia. However, the subject is not limited thereto and may include a variety of subjects with bacteremia or its complications.

The term "appropriateness of antibiotic therapy" as used herein refers to assessing whether an empirical antibiotic administered to a subject is an appropriate antibiotic, as it refers to assessing whether the administered antibiotic is reducing or inhibiting from proliferation of pathogenic organisms.

For example, appropriate may include cases in which the administered antibiotic is appropriate to relieve symptoms or to show all reactions associated with a favorable prognosis. On the other hand, inappropriate may mean a case where the administered antibiotic is inappropriate, resulting in insufficient symptom relief or relatively poor prognosis.

The term "probability of appropriate antibiotic use" as used herein refers to percentage of the appropriateness of antibiotic therapy, with a value closer to 100 indicating appropriate and a value closer to 0 indicating inappropriate.

The term "time-series data" as used herein refers to data observed in a continuous flow of time. The time-series data is classified into univariate data, bivariate data, or multi-variate data depending on the number of variables.

For example, when three or more variables are analyzed simultaneously in time-series analysis, it is called multivariable time-series analysis, which is used to understand the interaction and influence between variables.

The term "bio-signal data" as used herein may refer to all data associated with blood pressure, pulse, respiratory rate, and body temperature that may be obtained from a subject.

According to another feature of the present invention, the bio-signal data may include at least one of systolic blood pressure (SBP), diastolic blood pressure (DBP), body temperature, heart rate, and respiratory rate, but not limited thereto.

Preferably, the bio-signal data may be systolic blood pressure (SBP), diastolic blood pressure (DBP), body temperature, heart rate, and respiratory rate. For example, the bio-signal data may include a baseline value before administration of antibiotics and a value obtained at a time point within 5 days after administration of antibiotics in pairs. However, this is not limited thereto.

The term "blood test data" as used herein may refer to all data related to blood obtainable from a subject.

According to another feature of the present invention, the blood test data may include at least one of an aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels, but not limited thereto.

Preferably, the blood test data may be an aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels. For example, the blood test data may include a baseline value before administration of antibiotics and a value obtained at a time point within 5 days after administration of antibiotics in pairs. However, this is not limited thereto.

The term "clinical characteristic data" as used herein may mean characteristics of a subject, clinical evaluation, or analysis data of a biological sample (e.g., blood, cells, urine, etc.) separated from the subject.

According to another feature of the present invention, the clinical characteristic data may include at least one of the following: age, gender (Sex), Charlson Comorbidity Index score (CCI_score), PittScore, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicil-lin/clavulanate treatment status, or Ampicillin/sulbactam treatment status, but not limited thereto.

Preferably, according to another feature of the present invention, the clinical characteristic data may be age, gender (Sex), Charlson Comorbidity Index score (CCI_score), PittScore, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), cancer, or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicil-lin/clavulanate treatment status, or Ampicillin/sulbactam treatment status. However, this is not limited thereto.

The terms "the prediction model" as used herein may be a model configured to output appropriateness of antibiotic use using bio-signal data, blood test data, and clinical characteristic data obtainable from the subject as input.

According to a feature of the present invention, the prediction model may be a model trained by a step of predicting the appropriateness of antibiotic use based on training data consisting of bio-signal data, blood test data, and clinical characteristic data on a blood-infected subject to which an appropriate antibiotic is administered and a blood-infected subject to which an inappropriate antibiotic is administered.

According to another feature of the present invention, the prediction model may be any one selected from the group consisting of convolutional neural networks (CNN), deep neural networks (DNN), and recurrent neural networks (RNN), but not limited thereto.

According to another feature of the present invention, the recurrent neural network (RNN) may be a long short-term memory (LSTM) or a gated recurrent unit (GRU), but not limited thereto.

Meanwhile, the prediction model may be based on at least one algorithm of a logistic regression (LR)-based model, a random forest (RF)-based model, an extreme gradient boosting (XGB)-based model, and a deep learning model (DLM), but not limited thereto.

For example, the prediction model may be based on at least one algorithm selected from among U-net, VGG net, DenseNet, and a Fully Convolutional Network (FCN) with an encoder-decoder structure, a deep neural network (DNN) such as SegNet, DeconvNet, DeepLAB V3+, SqueezeNet, Alexnet, ResNet18, MobileNet-v2, GoogLeNet, Resnet-v2, Resnet50, RetinaNet, Resnet101, and Inception-v3. Furthermore, the prediction model may be an ensemble model based on at least two of the aforementioned algorithms.

However, the present disclosure is not limited thereto, and the prediction model for appropriateness of antibiotic use used in various embodiments of the present disclosure may be based on a wider variety of algorithms as long as it predicts the appropriateness of antibiotic therapy for a subject.

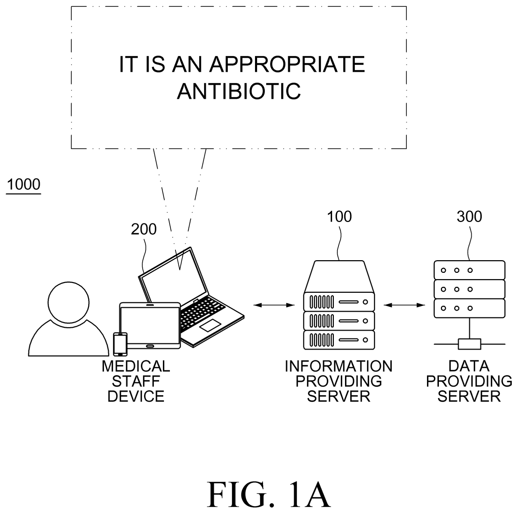

Hereinafter, a device for providing information on the appropriateness of antibiotic use and a system for providing information on the appropriateness of antibiotic use using the device according to an embodiment of the present invention will be described with reference to FIGS. 1A to 2B.

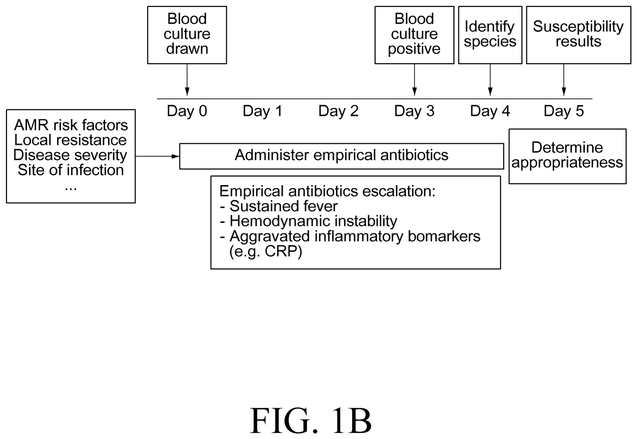

FIG. 1A illustrates a system for providing information on the appropriateness of antibiotic use using a device for providing information on the appropriateness of antibiotic use according to an embodiment of the present invention. FIG. 1B exemplarily illustrates a process of selecting an antibiotic based on a blood culture test to identify a pathogenic organism in a bacteremia patient.

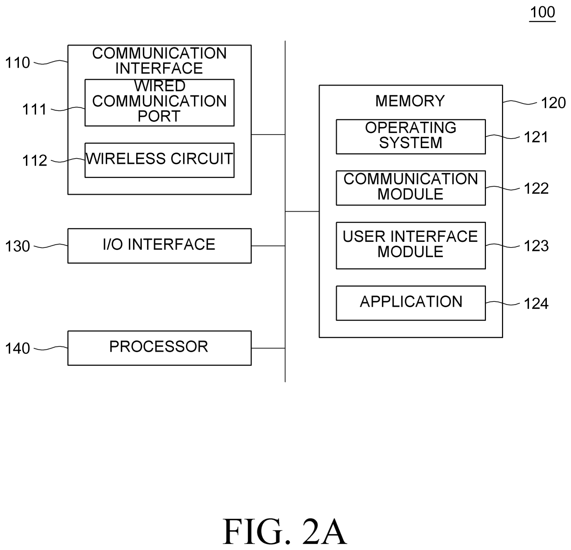

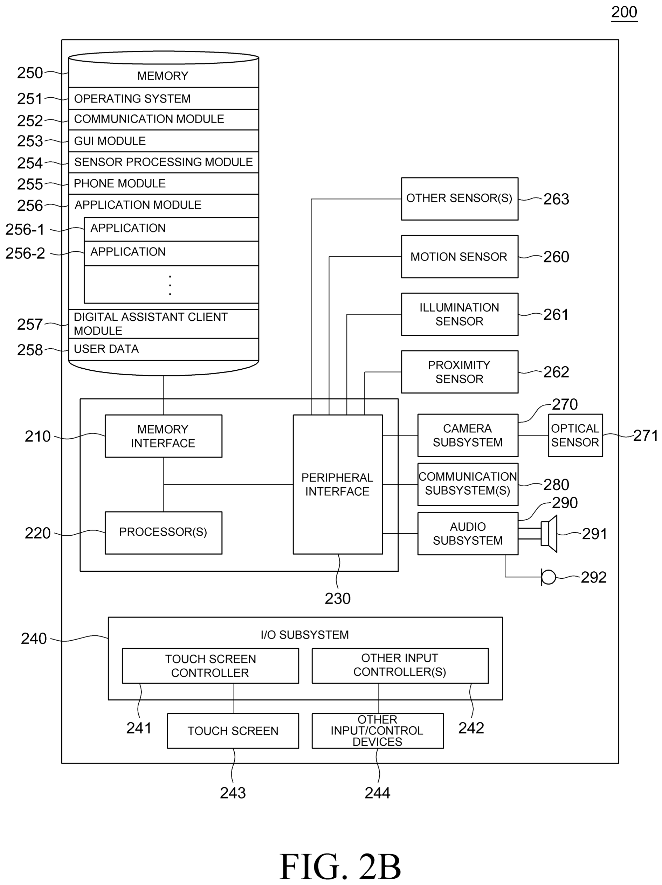

FIG. 2A exemplarily illustrates a configuration of an information providing server according to an embodiment of the present invention. FIG. 2B exemplarily illustrates a configuration of a medical staff device according to an embodiment of the present invention.

Referring to FIG. 1A, the information providing system 1000 may be a system configured to provide information related to appropriateness of antibiotic use based on bio-signal data, blood test data, and clinical characteristic data for a subject. In this case, the information providing system 1000 may include a medical staff device 200 that receives information associated with appropriateness of antibiotic use, a data providing server 300 that provides bio-signal data, blood test data, and/or clinical characteristic data, and an information providing server 100 that generates information about appropriateness of antibiotic use based on the received bio-signal data, blood test data, and clinical characteristic data.

First, the information providing server 100 may include a general-purpose computer, a laptop, and/or a data server that performs various operations for determining information associated with the appropriateness of antibiotic therapy based on bio-signal data, blood test data, and/or clinical characteristic data provided from the data providing server 200. In this case, the information providing server 100 may be a device for accessing a web server providing a web page or a mobile web server providing a mobile web site, but not limited thereto.

In this case, the information providing server 100 may comprise at least one prediction model (not shown) configured to predict the appropriateness of antibiotic use using bio-signal data, blood test data, and/or clinical characteristic data as input. Here, at least one prediction model is pre-learned, and the prediction model may be a convolutional neural network (CNN), a deep neural network (DNN), or a recurrent neural network (RNN). However, as another embodiment, a prediction model may be stored in a separate data learning server (not shown), and the information providing server 100 may be linked with the data learning server.

More specifically, the information providing server 100 may receive bio-signal data, blood test data, and/or clinical characteristic data from the data providing server 300, and determine appropriateness of antibiotic use for the subject based on the received bio-signal data, blood test data, and/or clinical characteristic data to provide information associated with the appropriateness of antibiotic therapy. In this case, the information providing server 100 may predict information associated with the appropriateness of the antibiotic therapy for the subject from the bio-signal data, the blood test data, and/or the clinical characteristic data using the prediction model.

Based on the bio-signal data, blood test data, and/or clinical characteristic data, the information providing server 100 may provide a prediction result for the appropriateness of antibiotic therapy using a prediction model, and preferably provide the result value in the form of whether the empirical antibiotic administered to the subject is appropriate(e.g., appropriate/inappropriate, YES/NO or O/X). However, it is not limited thereto and may be provided in the form of a probability value such as an probability of appropriate antibiotic use (e.g., 75% or 0.75).

The information providing server 100 may receive clinical characteristic data and bio-signal data and blood test data, which include a baseline value before administration of antibiotics and a value obtained at a time point within 5 days after administration of antibiotics, as pairs, from the data providing server 300.

More specifically, the information providing server 100 may receive clinical characteristic data and bio-signal data and blood test data, which include a baseline value before administration of antibiotics and a value obtained at a time point within 5 days after administration of antibiotics, as pairs, from the data providing server 300, and predict information associated with the appropriateness of antibiotic use administered to subject by using a prediction model configured to output a result of the appropriateness of antibiotic use for the subject based on the received bio-signal data, blood-test data, and clinical characteristic data.

Meanwhile, the bio-signal data and the blood test data may be time-series data collected from the subject for 5 days after administration of antibiotics. In this case, when the bio-signal data, blood test data, and clinical characteristic data are obtained at a first time point, the appropriateness of antibiotic therapy for the subject may be defined as the appropriateness for the first time point. Here, the first time point means a time point within 5 days after antibiotic treatment.

Furthermore, the information providing server 100 may be further configured to re-receive bio-signal data, blood test data, and clinical characteristic data obtained from the subject at a second time point, in this case, the information providing server 100 may be further configured to predict the appropriateness of antibiotic therapy for the subject based on the re-received bio-signal data, blood test data, and clinical characteristic data, and the appropriateness of antibiotic therapy for the subject may be defined as appropriateness for the second time point. Here, the second time point means another time point between the first time point and 5 days after antibiotic treatment.

The information providing server 100 may provide a prediction result associated with the appropriateness of antibiotic therapy for the subject to the medical staff device 200.

Next, the medical staff device 200 may receive a prediction result associated with the appropriateness of the antibiotic therapy for the object from the information providing server 100, and display the received result through a display device (e.g., a touch screen, a monitor, a projector, etc.).

The information provided from the information providing server 100 may be provided as a web page through a web browser installed in the medical staff device 200, or may be provided in the form of an application or a program. In various embodiments, such data may be provided in a form included in a platform in a client-server environment.

Meanwhile, a medical staff device 200 may be a computer capable of installing and executing a plurality of applications required by medical staff, an ultra-mobile PC (UMPC), a workstation, a net-book, personal digital assistants (PDA), a portable computer, a web tablet, a wireless telephone Mobile phone, smart phone, pad, smart watch, wearable terminal, e-book, portable multi-media player (PMP), portable game console, navigation device, black box or digital camera, other mobile communication terminals may be used, but are not limited to the aforementioned terminals.

Referring to FIG. 1B, it takes about 5 days to determine an optimal appropriate antibiotic based on the results after the bacterial identification test is performed. Specifically, in order to determine the optimal antibiotics for bacteremia patients, blood culture tests are performed to identify pathogenic organism, and blood culture tests are performed in the following order: blood collection, blood culture, confirmation of bacterial infection, bacterial identification, and antibiotic susceptibility tests. At this time, after the blood culture, the results of confirming bacterial infection on the 3rd day, the results of the bacterial species identification on the 4th day, and the results of the antibiotic susceptibility test on the 5th day are reported, and the medical team determines an appropriate antibiotic for the patient based on the results of the antibiotic susceptibility test.

On the other hand, it is common to use empirical antibiotics in consideration of the patient's age, history of infection diagnosis, and clinical symptoms before determining appropriate antibiotics by identifying the exact pathogenic organism based on the results of blood culture tests. However, at this time, one in four cases is prescribed inappropriate antibiotics that do not fit the patient's condition, and this inappropriate use has been reported to not only lead to serious side effects such as the development of antibiotic-resistant bacteria and worsening symptoms, but also to be closely associated with an increased risk of death.

Referring back to FIG. 1A, on the other hand, an information providing system 1000 for appropriateness of antibiotic use can predict information associated with the appropriateness of antibiotic therapy for a subject based on bio-signal data, blood test data, and clinical characteristic data obtained from a subject within 5 days of antibiotic treatment by using an antibiotic use appropriateness prediction model, thereby allowing medical staff to treat a subject in need of antibiotic administration while waiting for a bacterial identification test result.

Next, components of the information providing server 100 according to the present invention will be described in detail with reference to FIG. 2A.

Referring to FIG. 2A, the information providing server 100 may include a communication interface 110, a memory 120, an I/O interface 130, and a processor 140, and each component may communicate with each other through one or more communication buses or signal lines. Meanwhile, although not illustrated in FIG. 2A, a prediction model may be stored and stored as a module or stored in a separate data learning server (not shown) linked with the information providing server 100.

The communication interface 110 may be connected to the medical staff device 200 and the data providing server 300 through a wired/wireless communication network to exchange data. For example, the communication interface 110 may receive bio-signal data, blood test data, and clinical characteristic data from the data providing server 300 and transmit information associated with the determined appropriateness of antibiotic use to the medical staff device 200.

Meanwhile, a communication interface 110 enabling transmission and reception of such data includes a wired communication port 111 and a wireless circuit 112, wherein the wired communication port 111 may include one or more wired interfaces, for example, Ethernet, a universal serial bus (USB), a Firewire, and the like. In addition, the wireless circuit 112 may transmit and receive data to and from an external device through an RF signal or an optical signal. In addition, wireless communication may use at least one of a plurality of communication standards, protocols and technologies, such as GSM, EDGE, CDMA, TDMA, Bluetooth, Wi-Fi, VoIP, Wi-MAX, or any other suitable communication protocol.

The memory 120 may store data on at least one process (algorithm) for providing a broadcast service or a program reproducing the process. In addition, the memory 120 may further store processes for performing other operations, but this is not limited thereto.

The memory 120 may store various data used in the information providing server 100. For example, the memory 120 may store bio-signal data, blood test data, and/or clinical characteristic data, or may store a prediction model trained to predict appropriateness of antibiotic use for a subject based on the bio-signal data, blood test data, and/or clinical characteristic data.

In various embodiments, memory 120 may include a volatile or nonvolatile recording medium capable of storing various data, commands, and information. For example, the memory 120 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain database.

In various embodiments, the memory 120 may store the configuration of at least one of the operating system 121, the communication module 122, the user interface module 123, and one or more applications 124.

The operating system 121 (e.g., a built-in operating system such as LINUX, UNIX, MAC OS, WINDOWS, VxWorks, etc.) may include various software components and drivers for controlling and managing general system tasks (e.g., memory management, storage device control, power management, etc.), and may support communication between various hardware, firmware, and software components.

The communication module 122 may support communication with other devices through the communication interface 110. The communication module 122 may include various software components for processing data received by the wired communication port 111 or the wireless circuit 112 of the communication interface 110.

The user interface module 123 may receive a viewer's request or input from a keyboard, a touch screen, a microphone, etc. through the I/O interface 130, and provide a user interface on the display.

Application 124 may include programs or modules configured to be executed by one or more processors 140.

The I/O interface 130 may connect at least one of an input/output device (not shown) of the information providing server 100, for example, a display, a keyboard, a touch screen, and a microphone, to the user interface module 123. The I/O interface 130 may receive a viewer's input (e.g., voice input, keyboard input, touch input, etc.) together with the user interface module 123 and process a command according to the received input.

The processor 140 may be connected to the communication interface 110, the memory 120, and the I/O interface 130 to control the overall operation of the information providing server 100 and perform various commands through an application or a program stored in the memory 120.

The processor 140 may correspond to a computing device such as a central processing unit (CPU) or an application processor (AP). In addition, the processor 140 may be implemented in the form of an integrated chip (IC) such as a system on chip (SoC) in which various computing devices are integrated. Alternatively, the processor 140 may include a module for computing an artificial neural network model, such as a neural processing unit (NPU).

In various embodiments, the processor 140 may be configured to use at least one prediction model to predict the appropriateness of antibiotic therapy for the subject, particularly whether the antibiotic administered to the subject is appropriate, based on bio-signal data, blood test data, and clinical characteristic data.

The processor 140 can provide a predictive result of the appropriateness of antibiotic therapy using a prediction model based on the bio-signal data, blood test data, and/or clinical characteristic data, and preferably provide the result value in the form of whether the empirical antibiotic administered to the subject is appropriate (e.g., appropriate/inappropriate, YES/NO, or O/X). However, it is not limited thereto and may be provided in the form of a probability value such as an probability of appropriate antibiotic use (e.g., 75% or 0.75).

Based on the bio-signal data, blood test data, and clinical characteristic data, the prediction model can provide a predictive result of the appropriateness of antibiotic therapy as an output value, and preferably, whether the empirical antibiotic administered to the subject is appropriate(e.g., appropriate/inappropriate, YES/NO or O/X) as an output value. However, the output value is not limited thereto and the prediction model can also determine the probability of appropriate antibiotic use, thereby providing an probability of appropriate antibiotic use (e.g., 75% or 0.75).

Next, components of the medical staff device 200 of the present invention will be described in detail with reference to FIG. 2B.

Referring to FIG. 2B, the medical staff device 200 may include a memory interface 210, one or more processors 220, and a peripheral interface 230. Various components within medical staff device 200 may be connected by one or more communication buses or signal lines.

The memory interface 210 may be connected to the memory 250 to transmit various data to the processor 220. Here, the memory 250 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain database.

In various embodiments, the memory 250 may store at least one of an operating system 251, a communication module 252, a graphical user interface module (GUI) 253, a sensor processing module 254, a telephone module 255, and an application module 256. Specifically, the operating system 251 may include instructions for processing a basic system service and instructions for performing hardware tasks. Communication module 252 may communicate with at least one of one or more other devices, computers, and servers. The graphic user interface (GUI) module 253 may process a graphic user interface. The sensor processing module 254 may process sensor-related functions (e.g., processing voice input received through one or more microphones 292). The telephone module 255 may process telephone-related functions. The application module 256 may perform various functions of a user application, such as electronic messaging, web browsing, media processing, browsing, imaging, and other process functions. In addition, the medical staff device 200 may store one or more software applications 256-1, 256-2 (e.g., an information providing application) associated with any one type of service in the memory 250.

In various embodiments, a memory 250 may store a digital assistant client module 257 (hereinafter, referred to as a DA client module), and accordingly, may store instructions for performing functions on the client side of the digital assistant and various user data 258 (e.g., user customized vocabulary data, preference data, other data such as a user's electronic address book, etc.).

Meanwhile, the DA client module 257 may obtain a user's voice input, text input, touch input, and/or gesture input through various user interfaces (e.g., the I/O subsystem 240) provided in the medical staff device 200.

In addition, the DA client module 257 may output audio-visual and tactile types of data. For example, the DA client module 257 may output data composed of a combination of at least two of voice, sound, notifications, text messages, menus, graphics, video, animation, and vibration. In addition, the DA client module 257 may communicate with a digital assistant server (not shown) using the communication subsystem 280.

In various embodiments, the DA client module 257 may collect additional information about the surrounding environment of the medical staff device 200 from various sensors, subsystems, and peripheral devices to configure a context associated with user input. For example, the DA client module 257 may infer a user's intention by providing context information together with a user input to the digital assistant server. Here, the context information that may be accompanied by a user input may include sensor information, for example, light, ambient noise, ambient temperature, images of the surrounding environment, video, and the like. For another example, the context information may include a physical state of the medical staff device 200 (e.g., device orientation, device location, device temperature, power level, speed, acceleration, motion pattern, cellular signal strength, etc.). As another example, the context information may include information related to a software state of the medical staff device 200 (e.g., a process running on the medical device 200, an installed program, past and current network activity, background services, error logs, resource usage, etc.).

In various embodiments, memory 250 may include additional or deleted instructions. Furthermore, the medical staff device 200 may include additional components in addition to the components shown in FIG. 2B, or may exclude some components.

The processor 220 may control the overall operation of the medical staff device 200, and may execute various commands for implementing an interface provided by the information providing server 100 by running an application or a program stored in the memory 250.

The processor 220 may correspond to a computing device such as a central processing unit (CPU) or an application processor (AP). In addition, the processor 220 may be implemented in the form of an integrated chip (IC) such as a System on Chip (SoC) in which various computing devices for performing machine learning, such as a Neural Processing Unit (NPU) are integrated.

In various embodiments, the processor 220 may receive or request provision of various notifications, data, information, and the like through a user interface screen.

The peripheral interface 230 may be connected to various sensors, subsystems, and peripheral devices to provide data so that the medical staff device 200 may perform various functions. Here, the function performed by the medical device 200 can be understood as being performed by the processor 220.

The peripheral interface 230 may receive data from the motion sensor 260, the illumination sensor (optical sensor) 261, and the proximity sensor 262 so that the medical staff device 200 may perform orientation, light, and proximity sensing functions. For another example, the peripheral interface 230 may receive data from other sensors 263 (positioning system-GPS receiver, temperature sensor, biometric sensor), which may allow the medical staff device 200 to perform functions related to other sensors 263.

In various embodiments, the medical staff device 200 may include a camera subsystem 270 connected with the peripheral interface 230 and an optical sensor 271 connected thereto, such that the medical staff device 200 may perform various photographing functions such as taking pictures and recording video clips.

In various embodiments, the medical staff device 200 may include a communication subsystem 280 connected to the peripheral interface 230. The communication subsystem 280 may comprise one or more wired/wireless networks and may include various communication ports, radio frequency transceivers, and optical transceivers.

In various embodiments, a medical staff device 200 includes an audio subsystem 290 connected with a peripheral interface 230, and such audio subsystem 290 includes one or more speakers 291 and one or more microphones 292, such that the medical staff device 200 may perform voice-activated functions, such as voice recognition, voice replication, digital recording, and telephone functions.

In various embodiments, the medical staff device 200 may include an I/O subsystem 240 connected to the peripheral interface 230. For example, the I/O subsystem 240 may control the touch screen 243 included in the medical staff device 200 through the touch screen controller 241.

For example, the touch screen controller 241 may detect user contact and movement, or cessation of contact and movement, using any one of a number of touch sensing technologies, such as capacitive, resistive, infrared, surface acoustic wave, or proximity sensor arrays.
As another example, the I/O subsystem 240 may control other input/control devices 244 included in the medical staff device 200 through other input controller(s) 242. As an example, the other input controller(s) 242 may control pointer devices such as one or more buttons, rocker switches, a thumb-wheel, an infrared port, a USB port, and a stylus.

Referring to FIGS. 1A and 2A, it has been described that a method of providing information on the appropriateness of antibiotic use according to an embodiment of the present invention is performed in an information providing server and the result is displayed in a medical staff device, but not limited thereto. That is, the method of providing information on the appropriateness of antibiotic use according to an embodiment of the present invention may be configured to operate in the medical staff device itself. In this case, the operations described above may be performed by the processor 220.

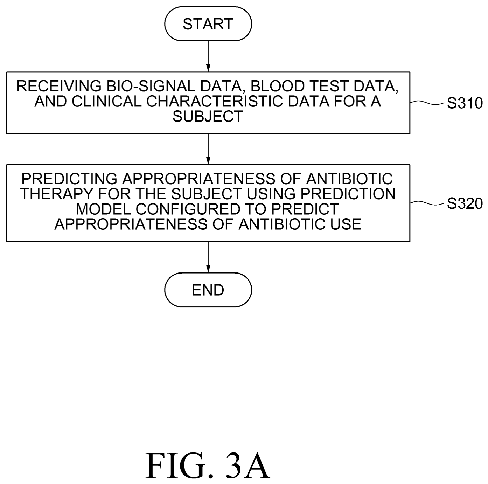

Hereinafter, a method of providing information on the appropriateness of antibiotic use according to an embodiment of the present invention will be described in detail with reference to FIGS. 3A to 3C. FIGS. 3A to 3C are flowcharts schematically illustrating a method of providing information on the appropriateness of antibiotic use according to an embodiment of the present invention.

Referring to FIG. 3A, an information providing procedure according to an embodiment of the present invention is as follows. First, bio-signal data, blood test data, and clinical characteristic data for a subject are received S310. Then, the appropriateness of antibiotic therapy for the subject is predicted by the prediction models320.

More specifically, the step S310 in which bio-signal data, blood test data, and clinical characteristic data for said subject are received and the step S320 in which the appropriateness of antibiotic therapy for the subject is predicted may be performed before results of a bacterial identification test for a subject having or suspected of having bacteremia are reported.

Referring to FIG. 1B, a medical team performs a bacterial identification test on a subject who has bacteremia or is suspected of having bacteremia, and then preemptively treats empirical antibiotics by evaluating the possibility of bacterial infection until the results of the bacterial identification test are released.

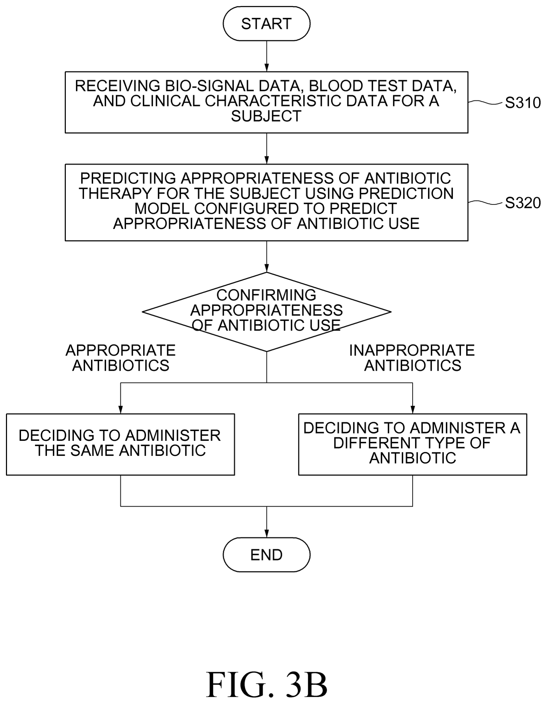

Referring to FIG. 3B, a medical staff may determine whether to change an antibiotic to be treated later by referring to information on the appropriateness of antibiotic therapy predicted by a prediction model for appropriateness of antibiotic use that provides information related to the appropriateness of antibiotic therapy for the subject as an output value, based on the bio-signal data, blood test data, and clinical characteristic data obtained from a subject. For example, if an empirical antibiotic treated preemptively is predicted to be an appropriate antibiotic, a decision can be made to continue administering the same antibiotic. Conversely, if the empirical antibiotic treated preemptively is predicted to be an inappropriate antibiotic, a decision can be made to administer a different antibiotic.

According to a feature of the present invention, the subject may be a patient with bacteremia, preferably a patient with acute severe bacteremia.

According to another feature of the present invention, the bacteremia may be a disease caused by at least one member selected from the group consisting of the species Staphylococcus aureus, the genus Streptococcus, the genus Enterococcus, the order Enterobacterales, the genus Acinetobacter, and the genus Pseudomonas.

More specifically, in a step S310 in which bio-signal data, blood test data, and clinical characteristic data for a subject are received, bio-signal data, blood test data, and clinical characteristic data for the subject are received at a first time point, and the first time point may be a time point before a bacterial identification test result is reported after antibiotic treatment, and preferably, may be a time point within 5 days after antibiotic treatment.

According to another feature of the present invention, the bio-signal data and the blood test data received S310 in a step, in which the bio-signal data, the blood test data, and the clinical characteristic data are received, may include, in pairs, a baseline value before administration of antibiotics and a value obtained at the first time point.

For example, bio-signal data, blood test data, and clinical characteristic data for the subject may be received at a predetermined time point after a certain period of time has elapsed following treatment with empirical antibiotics for the subject with or suspected of having bacteremia. The bio-signal data and the blood test data may include both a baseline value before taking empirical antibiotics and a value obtained at a time point after antibiotic treatment. At this time, the time point that a certain period of time has elapsed after empirical antibiotic treatment is the time point before the results of the bacterial identification test are reported, preferably within 24 hours after antibiotic treatment, in order to provide optimal treatment for subjects suspected of having bacteremia or having bacteremia.

According to another feature of the present invention, the bio-signal data received in step S310 in which the bio-signal data, the blood test data, and the clinical characteristic data are received may be at least one of a systolic blood pressure (SBP), a diastolic blood pressure (DBP), a body temperature, a heart rate, and a respiratory rate.

According to another feature of the present invention, the blood test data received in step S310 in which the bio-signal data, the blood test data, and the clinical characteristic data are received may be at least one of an aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels.

According to another feature of the present invention, the clinical characteristic data received at the step S310 in which bio-signal data, blood test data, and clinical characteristic data are received may beat least one of the following: age, gender (Sex), Charlson Comorbidity Index score (CCI_score), PittScore, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), cancer, or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicillin/clavulanate treatment status, or Ampicillin/sulbactam treatment status.

Next, in the step S320 of predicting the appropriateness of antibiotic therapy for the subject, the appropriateness of antibiotic therapy for the subject is determined based on the bio-signal data, blood test data, and clinical characteristic data received using the prediction model.

According to a feature of the present invention, in the step S320 of predicting the appropriateness of antibiotic therapy for the subject, the prediction model may predict whether the antibiotic administered to the subject is appropriate based on the bio-signal data, blood test data, and clinical characteristic data.

According to another feature of the present invention, the prediction model may be any one selected from the group consisting of convolutional neural networks (CNN), deep neural networks (DNN), and recurrent neural networks (RNN), but not limited thereto.

According to another feature of the present invention, the recurrent neural network (RNN) may be a long short-term memory (LSTM) or a gated recurrent unit (GRU), but not limited thereto.

According to another feature of the present invention, the prediction model may provide a prediction result for the appropriateness of antibiotic therapy as an output value based on the bio-signal data, blood test data, and clinical characteristic data, and preferably provide the probability for the appropriateness of antibiotic use determined by the prediction model as an output value.

More specifically, in a step S320 in which the appropriateness of antibiotic therapy for a subject is predicted, the bio-signal data and the blood test data including a pair of baseline values before taking the antibiotic and values obtained at a time point within 5 days after taking the antibiotic, and the clinical characteristic data are input into the antibiotic use appropriateness prediction model.

For example, bio-signal data and blood test data including a pair of baseline values before administration of antibiotics and values obtained at a time point after a certain period of time following empirical antibiotic treatment, and clinical characteristic data for the subject may be provided in a prediction model configured to predict appropriateness of antibiotic use. At this time, the time point that a certain period of time has elapsed after empirical antibiotic treatment is the time point before the results of the bacterial identification test are reported, preferably within 24 hours after antibiotic treatment in order to provide optimal treatment for subjects suspected of having bacteremia or having bacteremia.

Next, the prediction model predicts the appropriateness of antibiotic therapy for subjects by determining the probability of appropriateness of antibiotic use through a process of evaluating the change trend of data measurement values obtained at a time point within 5 days after administration and the baseline value before administration based on the input data.

In this case, the prediction model may be a model learned through a step of receiving training data composed of bio-signal data, blood test data, and clinical characteristic data for a blood-infected entity to which an appropriate antibiotic is administered and a blood-infected entity to which an inappropriate antibiotic is administered, and a step of predicting the appropriateness of antibiotic use based on the training data.

According to another feature of the present invention, the prediction result generated by the prediction model may be post-processed and provided to the user with various forms of result values.

For example, based on the probability of appropriateness of antibiotic use determined by the prediction model, a cut-off value for whether the empirical antibiotic administered to the subject is appropriate can be set to provide in the form of Y/N (e.g., appropriate/inappropriate, YES/NO, or O/X). The result value is not limited thereto and may be provided in the form of an probability of appropriate antibiotic use (e.g., 75% or 0.75) based on the determined probability of appropriateness of antibiotic use.

According to another feature of the present invention, in the step S320 of predicting the appropriateness of antibiotic therapy for the subject, the appropriateness of antibiotic therapy may be defined as appropriateness for the first time point, and the first time point may be a time point before the result of bacterial identification test after antibiotic treatment is reported, preferably a time point within 5 days after antibiotic treatment.

On the other hand, the appropriateness of antibiotic therapy for the subject is not limited at one time point, and if the result of the bacterial identification test is before being reported, bio-signal data, blood test data, and clinical characteristic data are re-received several times without limitation to predict the appropriateness of antibiotic therapy for various points in succession through an antibiotic use appropriateness prediction model.

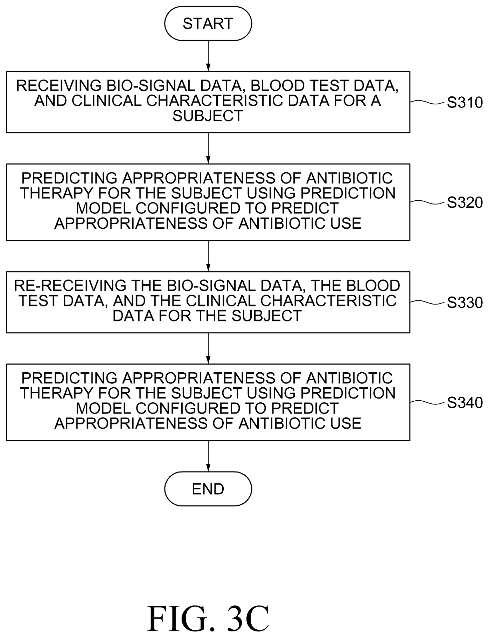

Accordingly, referring to FIG. 3C, after the step S310 of receiving the above-described bio-signal data, blood test data, and clinical characteristic data and the step S320 of predicting the appropriateness of antibiotic therapy for the subject by the prediction model, the step S330 of re-receiving the bio-signal data, the blood test data, and the clinical characteristic data for the subject and the step S340 of predicting the appropriateness of antibiotic therapy for the subject by the prediction model may be further performed.

According to another feature of the present invention, the bio-signal data and the blood test data may be time-series data collected from the subject for 5 days after administration of antibiotics, preferably data obtained in units of 6 to 24 hours for 5 days after administration of antibiotics, and more preferably, data obtained in units of 6 to 12 hours for 5 days after administration of antibiotics.

More specifically, in the step S330 of re-receiving the bio-signal data, blood test data, and clinical characteristic data for the subject, the bio-signal data, the blood test data, and the clinical characteristic data for the subject are re-received at a second time point, the second time point may be another time point between the first time point and a time point at which the bacterial identification test result is reported, and preferably, another time point between the first time point and 5 days after antibiotic treatment.

According to another feature of the present invention, the bio-signal data and blood test data received in step S330 of re-receiving the bio-signal data, blood test data, and clinical characteristic data may include a baseline value before administration of antibiotics and a value obtained at the second time point in pairs.

Next, in the step S340 of predicting the appropriateness of antibiotic therapy for the subject, the appropriateness of antibiotic therapy for the subject is determined based on the bio-signal data, blood test data, and clinical characteristic data re-received using the prediction model.

For example, baseline values and re-received bio-signal data, blood test data, and clinical characteristic data before administration of antibiotics can be provided in the LSTM model, which is configured to predict the appropriateness of antibiotic use.

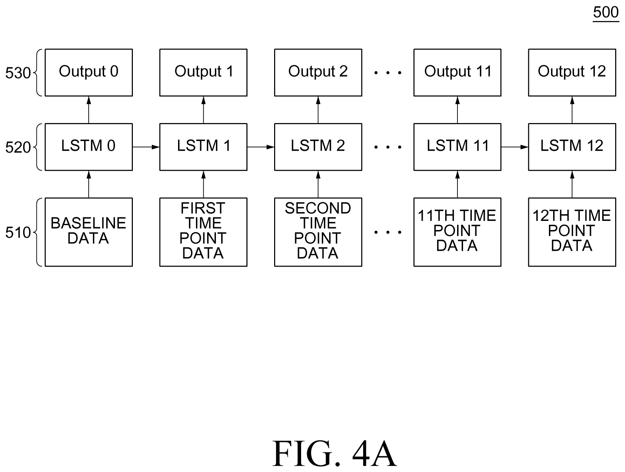

FIG. 4A exemplarily illustrates a structure of a time-series prediction model used in a method for providing information on the appropriateness of antibiotic use according to an embodiment of the present invention.

In the LSTM model, referring to FIG. 4A, the bio-signal data, blood test data, and clinical characteristic data may be provided to the input layer 510 of the LSTM model. In this case, the baseline value before taking the antibiotic is input as the baseline value data, the value obtained for the first time after the antibiotic treatment is input as the first time point data, and time-series data acquired after the first time point, such as the second time point data and the third time point data, may be sequentially input over time.

According to a feature of the present invention, in a step S340 in which the appropriateness of antibiotic therapy for a subject is predicted, based on bio-signal data, blood test data, and clinical characteristic data re-received by the prediction model, it is possible to predict whether the antibiotic administered to the subject is appropriate.

In this case, before the step S340 of predicting the appropriateness of the antibiotic therapy for the subject, the step S330-1 of further inputting the predicted result value of the step S320 of predicting the appropriateness of the antibiotic therapy described above in the prediction model may be further performed.

For example, based on the predicted result value of step S320 of predicting the appropriateness of antibiotic therapy by the prediction model described above and the re-received bio-signal data, blood test data, and clinical characteristic data, it is possible to predict whether the antibiotic administered to the subject is appropriate.

According to another feature of the present invention, in step S340 of predicting the appropriateness of antibiotic therapy for the subject, the appropriateness of antibiotic therapy may be defined as the appropriateness for the second time point.

According to another feature of the present invention, the prediction model may include a first prediction model configured to predict appropriateness of antibiotic use using the bio-signal data, blood test data, and clinical characteristic data obtained at the first time point as input, and a second prediction model configured to predict appropriateness of antibiotic use using the bio-signal data, blood test data, and clinical characteristic data obtained at the second time point as input. However, it is not limited thereto, and it is composed of one prediction model, and the same model may continuously yield new prediction result values.

According to another feature of the present invention, the prediction model may be any one selected from the group consisting of convolutional neural networks (CNN), deep neural networks (DNN), and recurrent neural networks (RNN), but not limited thereto. For example, when the prediction model is composed of two prediction models, the first prediction model may be a recurrent neural network (RNN), and the second prediction model may be a convolutional neural network (CNN). When the prediction model is composed of one prediction model, it may be a recurrent neural network (RNN) that uses the output of the previous time step as the input of the next time step for sequential prediction.

According to another feature of the present invention, the recurrent neural network (RNN) may be a long short-term memory (LSTM) or a gated recurrent unit (GRU), but not limited thereto.

Consequently, in the step S320 of predicting the appropriateness of antibiotic therapy for the subject, the appropriateness of antibiotic use for the subject within 5 days after antibiotic treatment can be predicted by the prediction model.

Accordingly, according to the information providing method according to various embodiments of the present invention, the medical team may be provided with information related to the appropriateness of antibiotic therapy for the subject, so that the optimal antibiotic therapy may be administered to the patient more quickly in the early stages of the infection in the treatment of bacteremia, where initial treatment is important.

In other words, by providing a system for providing information based on a prediction model trained to predict whether an antibiotic administered to a subject is an appropriate antibiotic, it can contribute to effective treatment of medical team even before the results of a bacterial identification test in a sample collected from blood or infected tissue are reported.

Therefore, it is possible to predict the appropriateness of antibiotic therapy regardless of the skill level of the medical staff, provide reliable information on the same, increase the survival rate of subjects with bacteremia, prevent complications, and reduce the cost of treatment.

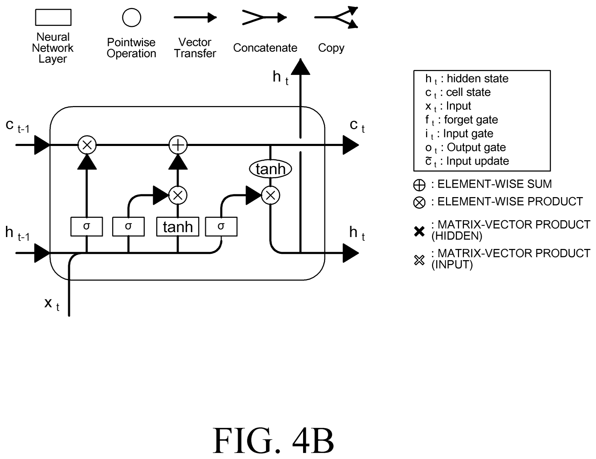

Hereinafter, referring to FIG. 4, structural features of an information prediction model associated with the appropriateness of antibiotic therapy for a subject used in various embodiments of the present invention will be described.

FIGS. 4A and 4B exemplarily illustrate the structure of a prediction model used in a method of providing information on the appropriateness of antibiotic use used in the method according to an embodiment of the present invention.

Referring to FIG. 4A, a prediction model 500 used in various embodiments of the present invention may comprise an input layer 510, an LSTM layer 520, and an output layer 530.

The input layer 510 is a first layer that receives input data provided from the outside and transmits the input data to a subsequent layer of the prediction model. Various types of data such as time-series data, text, and images are preprocessed into appropriate formats and transferred to subsequent layers.

The bio-signal data, blood test data, and clinical characteristic data obtained before administration of antibiotics and for 5 days after administration of antibiotics may be input into the input layer 510 in time series. In this case, the baseline value before taking the antibiotic is input as the baseline value data, the value obtained for the first time after the antibiotic treatment is input as the first time point data, and time-series data obtained after the first time point, such as the second time point data and the third time point data, may be sequentially input over time.

For example, the baseline value data may be baseline value data before administration of antibiotics, the first time point data may be data obtained at a time point within 5 days after treatment of antibiotics, the second time point data may be data obtained at another time point between the first time point and 5 days after treatment of antibiotics, and the third time point data may be data obtained at another time point between the second time point and 5 days after treatment of antibiotics.

The LSTM layer 520 is comprised of a long short-term memory (LSTM) unit, which is a type of recurrent neural network (RNN). The input data is processed to selectively store important information in each time step, remove unnecessary information, and thereby learn long-term dependencies of time-series data. In addition, by using the output of the previous time step as the input for the current time step, it maintains a temporal order and reflects the flow of data. That is, the LSTM layer 520 operates in a manner of transmitting the output of the previous time step as the input for the current time step to learn long-term dependencies.

The LSTM layer 520 is configured to predict the appropriateness of antibiotic therapy for the subject by using a time-series dataset and a time-invariant variable consist of a time-variant variable as input.

More specifically, time-series data are windowed at regular time intervals so that time-series patterns of bio-signal data and blood test data are input to a long short-term memory (LSTM) network learned in advance, and the prediction model extracts time-series features by reflecting long-term and short-term dependency. At the same time, the time-invariant variable is input to the prediction model through a separate path and combined with the time-series feature. Finally, the output of the LSTM cells and static variables are integrated in a Dense layer to convert into probability values, and then used to predict information related to the appropriateness of antibiotic therapy for the subject is ed.

Here, LSTM may solve the long-term dependency problem of RNN (Recurrent Neural Network), that is, the phenomenon in which the previous information is not properly transmitted to the current state over time if the length of the input sequence is long.

Referring to FIG. 4B, when information in each stage is stored in a cell state and sent to the next stage, how much the contents of the previous step will be forgotten (forget gate), how much the current input information will be accepted (input gate), and then the cell state to be stored in the current step using the information (input update gate), and the information to be outputted in a hidden state (output gate) is determined.

Referring back to FIG. 4A, the output layer 530 is the last layer that generates a final result of the prediction model. Based on the data processed in the LSTM layer, a final prediction value or classification result may be generated, and various types of outputs may be generated according to the purpose of the predicted model.

The output layer 530 may generate a prediction result for the appropriateness of antibiotic therapy as an output value, and preferably, a probability for the appropriateness of antibiotic use is determined as an output value. The generated output value may be provided to the processor to output the probability of appropriate antibiotic use (e.g., 75% or 0.75) as the final result, or whether the empirical antibiotic administered to the subject based on the cut-off value is appropriate (e.g., appropriate/inappropriate, YES/NO, or O/X) as the final result.

### Evaluation 1: Learning and evaluating prediction models used in various embodiments of the present invention

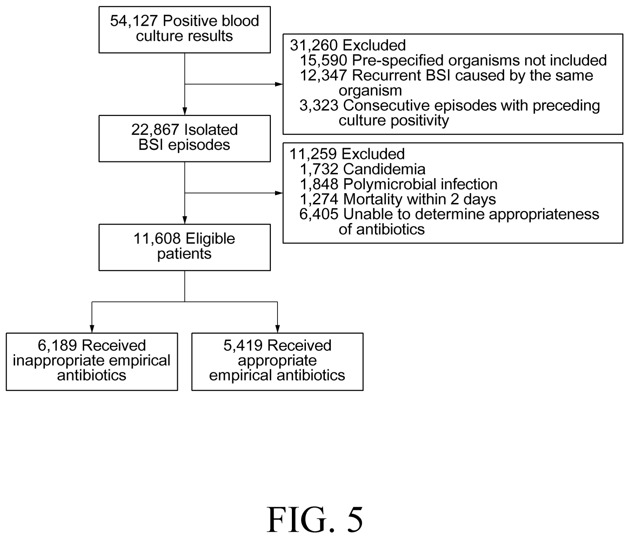

Hereinafter, the training data of the prediction model for appropriateness of antibiotic use and the training process of the prediction model used in various embodiments of the present invention will be described in detail with reference to FIGS. 5 to 7.

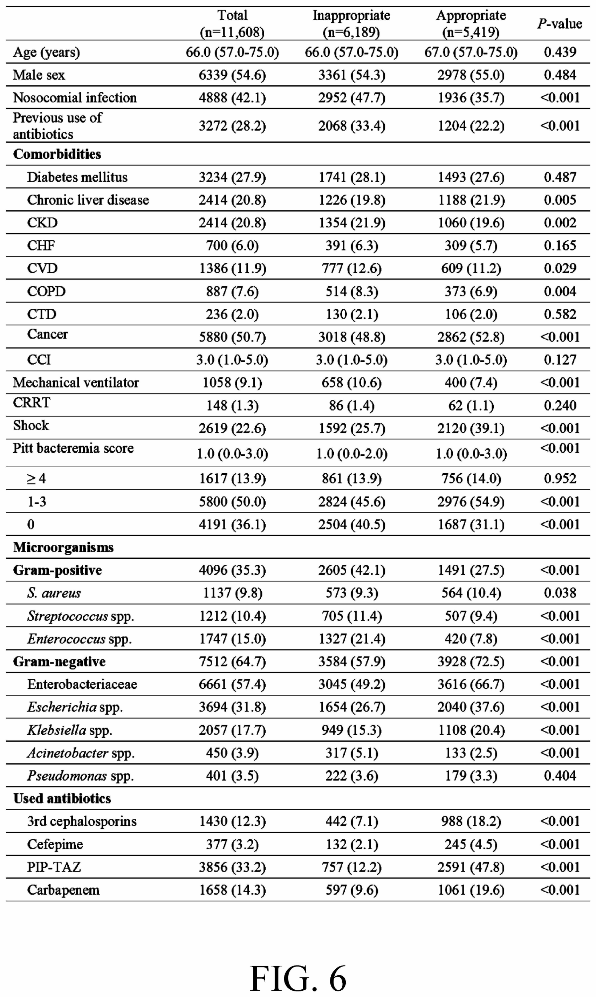

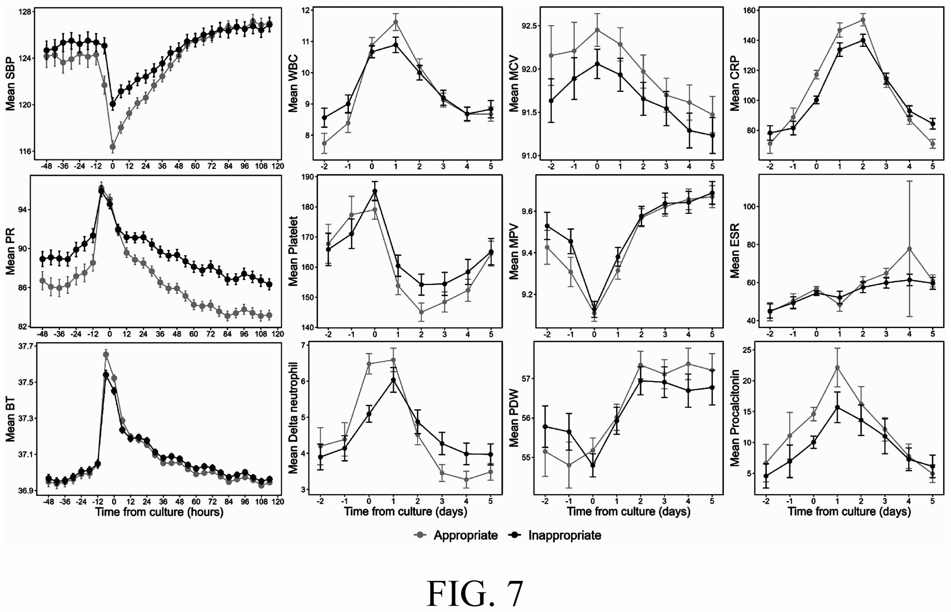

FIG. 5 illustrates a process of pre-processing and labeling training data of a prediction model for appropriateness of antibiotic use according to an embodiment of the present invention. FIG. 6 exemplarily illustrates training data according to an embodiment of the present invention. FIG. 7 illustrates the results of hemodynamic indicators and biomarker levels measured over time after antibiotic administration in patients administered appropriate antibiotics and patients administered inappropriate antibiotics, according to one embodiment of the present invention.

In this case, the prediction model may be a model trained to predict the appropriateness of antibiotic use based on bio-signal data, blood test data, and clinical characteristic data, but the training method thereof is not limited thereto. Furthermore, clinical data used to predict the appropriateness of antibiotic therapy may be selected in different configurations depending on the training method.

First, referring to FIG. 5, data of 54,127 patients with bloodstream infections (BSI) during hospitalization from Severance Hospital of Yonsei University were used for training and verifying the prediction model used in various embodiments of the present invention.

More specifically, training data obtained from sample subjects of a blood-infected subject to which an appropriate antibiotic is administered and a blood-infected subject to which an inappropriate antibiotic is administered may comprise bio-signal data, blood test data, and clinical characteristic data.

Here, the bio-signal data includes a systolic blood pressure (SBP), diastolic blood pressure (DBP), body temperature, heart rate, and respiratory rate.

Blood test data include an aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels.

Clinical characteristic data includes an age, gender (Sex), Charlson Comorbidity Index score (CCI_score), PittScore, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), cancer, or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicillin/clavulanate treatment status, or Ampicillin/sulbactam treatment status.

Referring back to FIG. 5, to classify single bloodstream infections (BSIs), infections other than those with pre-specified pathogens (15,590 cases), recurrent bloodstream infections by the same pathogens (12,347 cases), and previous culture-positive episodes as consecutive episodes (3,323 cases) were excluded.
The pre-specified strains are shown in Table 1 below.

**Table 1**

| Strain type | SPECIES |
|---|---|
| Gram-positive bacteria | *Staphylococcus aureus* |
| | *Streptococcus spp.* |
| | *Enterococcus spp.* |
| Gram-negative bacteria | *Enterobacterales (Escherichia spp., Klebsiella spp., Enterobacter spp., Citrobacter spp., etc.)* |
| | *Acinetobacter spp.* |
| | *Pseudomonas spp.* |

Next, from the 22,867 single bloodstream infection (BSI) cases, cases of candidemia (1,732 cases), polymicrobial infections (1,848 cases), death within 2 days (1,274 cases), and cases in which antibiotic appropriateness could not be determined (6,405 cases) were further excluded, and finally selected 11,608 patients as eligible, which were used as training data for the antibiotic appropriateness prediction model.

The data of 11,608 patients selected for eligibility were classified into patients who received inappropriate empirical antibiotic treatment (6,189) and those who received appropriate empirical antibiotic treatment (5,419) according to the appropriateness of empirical antibiotic treatment, where antibiotic appropriateness was defined as the administration of appropriate antibiotics if the antibiotic susceptibility was high for 3 days after blood culture.

Referring to FIG. 6, as a result of analyzing the clinical characteristics of patients with bacteremia, patients who received appropriate empirical antibiotic treatment were analyzed to have significantly lower nosocomial infection rates than patients who received inappropriate empirical antibiotic treatment, and the number of patients infected with Gram-positive bacteria and the number of patients infected with Gram-negative bacteria were 4,096 (35.5%) and 7,512 (64.7%), respectively.

Referring to FIG. 7, the changes in seven hemodynamics-related indicators (mean systolic blood pressure (Mean SBP), mean pulse rate (Mean PR), mean body temperature (Mean BT), mean white blood cell count (Mean WBC), mean corpuscular volume (Mean MCV), mean platelet volume (Mean MPV), mean platelet distribution width (Mean PDW), and five biomarkers (mean platelet count (Mean Platelet), mean delta Neutrophil, mean C-reactive protein (Mean CRP), mean erythrocyte sedimentation rate (Mean ESR), and mean procalcitonin) over time before and after antibiotic administration were examined, as a result, it was confirmed that there was a significant difference between the pattern of change in patients with appropriate empirical antibiotic treatment and the pattern of change in patients with inappropriate empirical antibiotic treatment for both hemodynamic-related indicators and biomarkers used in the analysis.

Based on the bio-signal data, blood test data, and clinical characteristic data, training data were constructed to learn changes in hemodynamic indicators and biomarkers over time in patients with appropriate empirical antibiotic treatment and those with inappropriate empirical antibiotic treatment, and utilized them to allow the deep learning model to learn the corresponding pattern of change to build a prediction model for early prediction of appropriateness of antibiotic use.

More specifically, the preprocessing of the training data, including missing value correction, was performed as follows. Only variables with a missing rate of less than 30% were included, and MissForest was used as the missing value correction method. In a single imputation method, the initial missing value was set as the mean for continuous variables and the mode for categorical variables. Thereafter, the random forest model was trained to predict the missing value, and these were then filled with the predicted value. This process was repeated until the missing values converged, and ultimately forming the training data.

As these 5 kinds of bio-signal data, 33 kinds of blood test data, 37 kinds of clinical characteristic data, and 38 kinds of baseline value before administration of antibiotics (5 kinds of bio-signal data and 33 kinds of blood test data) can be applied as input data to the training of the prediction model, in predicting the appropriateness of antibiotic therapy, the prediction model of the present invention may have better predictive ability of the appropriateness of antibiotic use than other models.

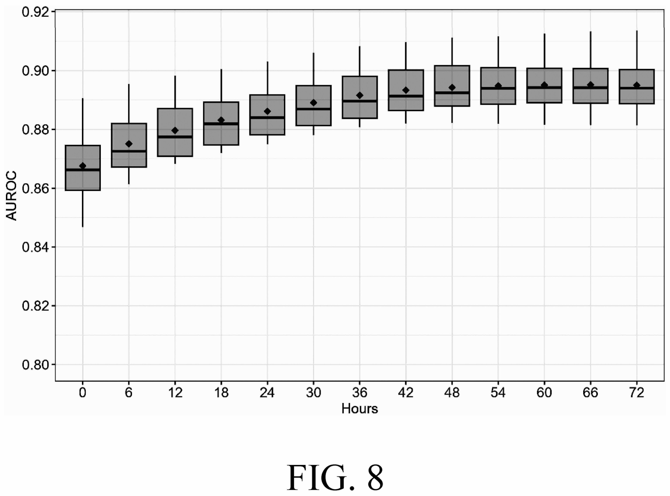

Hereinafter, the verification result of a model for predicting the appropriateness of antibiotic use used in various embodiments of the present invention will be described in detail with reference to FIG. 8.

FIG. 8 illustrates an AUROC analysis result of a model for predicting the appropriateness of antibiotic use over time according to an embodiment of the present invention.

Referring to FIG. 8, based on the aforementioned 5 kinds of bio-signal data, 33 kinds of blood test data, 37 kinds of clinical characteristic data, and 38 kinds of baseline value before administration of antibiotics (5 kinds of bio-signal data and 33 kinds of blood test data), evaluation results for predicting the appropriateness of antibiotic therapy in LSTM-based models configured to predict the appropriateness of antibiotic use are shown.

More specifically, in predicting the appropriateness of antibiotic use, it appears to have an AUROC of 0.886 (IQR (interquartile range), 0.871 to 0.888) at the time of 24 hours after the onset of bacteremia. In addition, as additional bio-signal data, blood test data, and clinical characteristic data are entered into the prediction model over time, a median AUROC of 0.895 (IQR, 0.889 to 0.901) appears at the time of 72 hours elapsed as the prediction model performs sequential predictions using time-series data.

In other words, these results may mean that the prediction model trained based on the bio-signal data, blood test data, and clinical characteristic data has excellent predictive performance for antibiotic therapy appropriateness when used before the bacterial identification test results are reported.

Although the embodiments of the present disclosure have been described in detail with reference to the accompanying drawings, the present disclosure is not limited thereto and may be embodied in many different forms without departing from the technical concept of the present disclosure. Therefore, the embodiments disclosed in the present disclosure are provided for illustrative purposes only but not intended to limit the technical spirit of the present disclosure. The scope of the technical spirit of the present disclosure is not limited thereby. Therefore, it should be understood that the above-described embodiments are illustrative in all aspects and do not limit the present disclosure. The protective scope of the present disclosure should be construed based on the following claims, and all the technical spirit in the equivalent scope thereto should be construed as falling within the scope of the present disclosure.

## Claims

1. A method for providing information on the appropriateness of antibiotic use implemented by a processor, comprising
receiving bio-signal data, blood test data, and clinical characteristic data for a subject; and in a first prediction step, predicting appropriateness of antibiotic therapy for the subject based on the received bio-signal data, blood test data, and clinical characteristic data using a prediction model configured to predict the appropriateness of antibiotic use with the bio-signal data, blood test data, and clinical characteristic data as input,
wherein the bio-signal data, blood test data, and clinical characteristic data for the subject are data obtained at a first time point,
wherein the first time point is within 5 days after antibiotic treatment,
wherein the appropriateness of antibiotic therapy for the subject in the first prediction step is defined as the appropriateness for the first time point.

2. The method of claim 1, wherein the subject is a patient with bacteremia.

3. The method of claim 1, wherein the bio-signal data and the blood test data are time-series data collected from the subject for 5 days after administration of antibiotics.

4. The method of claim 1, wherein the bio-signal data includes at least one of a systolic blood pressure (SBP), a diastolic blood pressure (DBP), a body temperature, a heart rate, and a respiratory rate, and
the bio-signal data includes a pair of baseline values before taking an antibiotic and a value obtained at a time point within 5 days after taking the antibiotic.

5. The method of claim 1, wherein the blood test data includes at least one of aPTT (Activated Partial Thromboplastin Time) level, a CRP (C-reactive Protein) level, an ESR (Erythrocyte Sedimentation Rate) level, a Lactate level, a Procalcitonin level, a PT/INR (Prothrombin Time/International Normalized Ratio) level, a Delta neutrophil level, Hematocrit level, Hemoglobin level, MCH (Mean Corpuscular Hemoglobin) level, MCHC (Mean Corpuscular Hemoglobin Concentration) level, MCV (Mean Corpuscular Volume) level, MPV (Mean Platelet Volume) level, PDW (platelet distribution width) level, PDW_fL level, PLT (platelet count) level, RBC (red blood cell count) level, RDW (red blood cell distribution width) level, TMA (thrombotic microangiopathy) score, WBC (white blood cell count) level, albumin level, ALP (alkaline phosphatase) level, ALT (alanine aminotransferase) level, AST (aspartate aminotransferase) level, bilirubin level, BUN (blood urea nitrogen) level, calcium level, cholesterol level, creatinine level, glucose level, Phosphate levels, total protein levels, and uric acid levels, and
the blood test data includes a pair of baseline values before taking an antibiotic and a value obtained at a time point within 5 days after taking the antibiotic.

6. The method of claim 1, wherein the clinical characteristic data includes at least one of the following: age, gender (Sex), Charlson Comorbidity Index score (CCI_score), PittScore, whether the subject suffered from diabetes mellitus, chronic liver disease, chronic kidney disease (CKD), congestive heart failure (CHF), cardiovascular disease (CVD), chronic obstructive pulmonary disease (COPD), connective tissue disease (CTD), cancer, or shock, or whether the subject is on ventilator support (Vent) status, continuous renal replacement therapy (CRRT) treatment status, inotropic drugs (Inotropics) treatment status, tigecycline treatment status, 1^{st} cephalosporins treatment status, 2^{nd} cephalosporins treatment status, 3^{rd} cephalosporins treatment status, 4^{th} cephalosporins treatment status, clindamycin treatment status, linezolid treatment status, Nafcillin treatment status, TMP-SMX treatment status, aminoglycoside treatment status, beta-lactam inhibitor treatment status, carbapenem treatment status, fluoroquinolones treatment status, glycopeptide treatment status, monobactam treatment status, penicillin treatment status, Polymyxin treatment status, Tetracycline treatment status, Piperacillin/tazobactam treatment status, Amoxicillin/clavulanate treatment status, or Ampicillin/sulbactam treatment status.

7. The method of claim 1, further comprising,
re-receiving a bio-signal data, blood test data, and clinical characteristic data obtained at a second time point; and
in a second prediction step, predicting the appropriateness of antibiotic therapy for the subject based on the re-received bio-signal data, blood test data, and clinical characteristic data, using the prediction model,
the second time point is another time point between the first time point and 5 days after antibiotic treatment,
the appropriateness of antibiotic therapy for the subject in the second prediction step is defined as appropriateness for the second time point, and
the re-received bio-signal data and blood test data includes a pair of baseline values before administration of antibiotics and a value obtained at the second time point.

8. **The** method of claim 8, wherein the prediction model comprises a first prediction model configured to predict the appropriateness of antibiotic use using the bio-signal data, blood test data, and clinical characteristic data obtained at the first time point as input, and a second prediction model configured to predict the appropriateness of antibiotic use using the bio-signal data, blood test data, and clinical characteristic data obtained at the second time point as input.

9. **The** method of claim 1, wherein the prediction model is model trained by receiving training data consisting of bio-signal data, blood test data, and clinical characteristic data obtained from a blood-infected subject to which an appropriate antibiotic is administered and a blood-infected subject to which an inappropriate antibiotic is administered, and predicting the appropriateness of antibiotic use based on the training data.

10. A device for providing information on the appropriateness of antibiotic use, comprising, a receiving unit configured to receive a bio-signal data, blood test data, and clinical characteristic data from a subject, and
a processor connected to the receiving unit, the processor is configured to predict the appropriateness of antibiotic therapy for the subject based on the received bio-signal data, blood test data, and clinical characteristic data, using a prediction model for appropriateness of antibiotic use configured to predict appropriateness of antibiotic use by taking the bio-signal data, blood test data, and clinical characteristic data as inputs, wherein the bio-signal data, blood test data, and clinical characteristic data is a data obtained at a first time point, the first time point being within 5 days of antibiotic treatment, and the appropriateness of antibiotic therapy for the subject is defined as appropriateness for the first time point.

11. **The** device of claim 10, wherein the subject is a patient with bacteremia.

12. **The** device of claim 10, wherein the bio-signal data and the blood test data are time-series data collected from the subject for 5 days after administration of antibiotics.

13. **The** device of claim 10, wherein the bio-signal data includes at least one of a systolic blood pressure (SBP), a diastolic blood pressure (DBP), a body temperature, a heart rate, and a respiratory rate, and
the bio-signal data includes a pair of baseline values before taking an antibiotic and a value obtained at a time point within 5 days after taking the antibiotic.

14. **The** device of claim 10, wherein the receiving unit is further configured to re-receive bio-signal data, blood test data, and clinical characteristic data obtained at a second time point from the subject, and
the processor is further configured to predict the appropriateness of antibiotic therapy for the subject based on the re-received bio-signal data, blood test data, and clinical characteristic data,
the second time point is another time point between the first time point and 5 days after antibiotic treatment, and
the appropriateness of antibiotic therapy for the subject is defined as appropriateness for the second time point.

15. **The** device of claim 14, wherein the prediction model for appropriateness of antibiotic use comprises a first prediction model configured to predict the appropriateness of antibiotic use using the bio-signal data, blood test data, and clinical characteristic data obtained at the first time point as input, and
a second prediction model configured to predict the appropriateness of antibiotic use using the bio-signal data, blood test data, and clinical characteristic data obtained at the second time point as input.
